# EUROPÄISCHE PATENTSCHRIFT

(11) **EP 1 239 809 B1**
(45) Veröffentlichungstag und Bekanntmachung des Hinweises auf die Patenterteilung: **20.08.2003**
(21) Anmeldenummer: 00982749.4
(22) Anmeldetag: 05.12.2000
(51) Int. Cl.: A61J 17/00

(54) **SCHNULLER FÜR FRÜHGEBORENE**
PACIFIER FOR A PREMATURE INFANT
SUCETTE POUR PREMATURE

(30) Priorität: 23.12.1999 AT 217999
(43) Veröffentlichungstag der Anmeldung: 18.09.2002
(73) Patentinhaber: Bamed AG, 8852 Altendorf (CH)
(72) Erfinder: RÖHRIG, Peter, A-1160 Wien (AT)
(74) Vertreter: Weinzinger, Arnulf, Dipl.-Ing.
(86) Internationale Anmeldenummer: AT0000328
(87) Internationale Veröffentlichungsnummer: WO01047467

(56) Entgegenhaltungen:
- DE-A- 3 728 668
- US-A- 5 078 733
- US-A- 5 546 938

## Beschreibung

Die Erfindung betrifft einen Schnuller für Frühgeborene mit einem Schild, an dem ein Sauger sowie vorzugsweise ein Griff, angebracht ist, und der mit zumindest einer Ausnehmung für die Nase versehen ist.

Bei medizinischen Untersuchungen hat sich gezeigt, dass Frühgeborene durch die Verwendung eines Schnullers besser trinken lernen und dadurch schneller an Gewicht aufholen. Die Benutzung eines Schnullers ist für Frühgeborene jedoch häufig problematisch, da die im Handel erhältlichen Schnuller zu groß für den Gebrauch durch Frühgeborene sind. Überdies sind Frühgeborene zu klein, um Nahrung mittels Flaschen zu sich zu nehmen, so dass Nahrungsmittel und auch Medikamente über Nasal- oder Mundintubationen zugeführt werden müssen, was Platzprobleme für das Einsetzen eines Schnullers in den Mund schafft. Ein weiteres Problem ist, dass die Schläuche durch den Schnuller gegen den Mund bzw. die Nase des Säuglings gedrückt werden und dies Schmerzen verursachen kann.

In der US 5 078 733 A ist ein Schnuller beschrieben, der einen Schild mit einer Ausnehmung für die Nase und gegebenenfalls für Nasalintubationen aufweist. Dies ermöglicht ein Einsetzen des Schnullers in den Mund eines Frühgeborenen, ohne eine Nasalintubation versetzen oder entfernen zu müssen. Die Mundintubation wird jedoch durch den Schild gegen den Mund des Frühgeborenen gedrückt, was für Frühgeborene, insbesondere wenn diese Mundintubation über eine längere Zeit hinweg gegen den Mund drückt, nachteilig ist.

Aus der WO 92/11836 ist ein Schnuller für Frühgeborene bekannt, wobei der Rand des Schildes des Schnullers eine Einbuchtung für die Nase aufweist. Der Schnuller hat kleinere Dimensionen als herkömmliche Schnuller, so dass auch verhindert wird, dass der Schnullersauger durch Anstoßen des Schildes auf einer Unterlage aus dem Mund des Säuglings geschoben wird, wenn sich dessen Kopf in der seitlichen Liegeposition befindet. Auch hier ist das Einsetzen von Mundintubationen jedoch problematisch.

Die US 4 796 628 A und die DE 37 28 668 A1 betreffen Schnuller, bei denen der Schnullerschild seitliche Ausnehmungen zur Aufnahme von Leitungen bzw. Schläuchen aufweist. Hier drückt der Schild jedoch gegen die Nase, und weiters ist es nachteilig, dass eine Einheitsgröße vorgesehen ist, so dass der Schnuller je nach Fall zu groß oder zu klein für den Säugling sein kann.

Es ist nun Ziel der Erfindung, einen Schnuller der eingangs angeführten Art vorzusehen, der für verschieden große Frühgeborene geeignet ist, wobei eine individuelle Anpassung an das jeweilige Kind möglich ist und Nasal- bzw. Mundintubationen problemlos ermöglicht werden.

Der erfindungsgemäße Schnuller der eingangs angeführten Art ist dadurch gekennzeichnet, dass der Schild abtrennbare Bereiche aufweist, denen als Schwächungsstellen oder Markierungen definierte, vorgegebene Trennstellen zugeordnet sind. Aufgrund der vom restlichen Schild, beispielsweise durch Abreißen, Abbrechen oder durch Abschneiden, abtrennbaren Bereiche, kann der Schnuller auf die jeweils erforderliche Größe "zurechtgestutzt" werden. Die Schnullerschilde werden durch das Abtrennen von Bereichen kleiner und handlicher. Die abtrennbaren Bereiche können je nach Situation alle abgetrennt werden, oder es werden nur die jeweils störenden Schildbereiche entfernt. Insbesondere werden Schnuller zur Verfügung gestellt, die bei der Zufuhr von Nahrungsmitteln, Luft, Medikamenten etc. durch Schläuche, die in die Nase oder in den Mund des Säuglings führen, individuell angepasst werden können und auch ausreichend Platz für Intubationen bieten. Dadurch wird andererseits die problemlose Verwendung von Schnullern bei Frühgeborenen ganz generell ermöglicht, was wiederum deshalb Vorteile erbringt, weil das Saugen an einem Schnuller insbesondere für unterentwickelte Frühgeborene vorteilhaft ist, da diese durch das Saugen eine stärkere Mundmuskulatur entwickeln und auch besser trinken lernen; dies führt wiederum zu einer raschen Gewichtszunahme. Die Schnullerschilde weisen z.B. ungefähre Abmessungen von 20-40 mm x 40-60 mm auf, und es können zusätzlich zu den vorgegebenen Trennstellen weitere Löcher vorgesehen sein, die eine Mundtrocknung und Hautatmung bzw. ein Not-Atmen des Kindes im Fall eines Verschluckens des Schnullers gewährleisten. Andererseits wird die Herstellung des Schnullers vereinfacht, da lediglich eine Einheitsgröße in Bezug auf die Schildgröße produziert wird.

Im Rahmen der vorliegenden Erfindung werden unter "Schwächungsstellen" Bereiche im Schnullerschild verstanden, die einen geringeren Reißwiderstand, z.B. zufolge einer geringeren Dicke oder von Perforationen, im Vergleich zum restlichen Schnullerschild aufweisen. Markierungen an den Trennstellen sind dann besonders vorteilhaft, wenn die gewünschte Größe des Schildes vorweg optisch festgelegt wird, und auch, wenn die abtrennbaren Bereiche mittels Schere oder Messer abgeschnitten werden, da dann ein exaktes Schneiden an den gewünschten Trennstellen erleichtert wird. Die Markierungen können in Form von im Vergleich zum restlichen Schnullerschild andersfärbigen Punkten, Linien, Schraffierungen etc. vorgesehen sein.

Es ist vorteilhaft, wenn ein bei der Benutzung im Nasenbereich liegender Randbereich des Schildes abtrennbar vorgesehen ist. Wird dieser obere Randbereich des Schildes abgetrennt, so wird dadurch Platz für Nasalintubationen geschaffen, so dass die Schläuche problemlos in die Nase eingeführt bzw. wieder herausgenommen werden können, ohne den Schnuller aus dem Mund des Kindes entfernen zu müssen, so dass diese Handlungen auch bei einem schlafenden Kind durchgeführt werden können. Schnuller sind gerade bei Nasalintubation sehr wichtig, da bei Nasalintubation automatisch eine Mundatmung eintritt, die für die Entwicklung des Säuglings nicht optimal ist. Durch das Saugen am Schnuller stellt der Säugling wieder auf die Nasenatmung um, die wiederum eine optimale Entwicklung des Frühgeborenen ermöglicht.

Vorzugsweise ist ein bei der Benutzung seitlich, im Mundwinkelbereich, liegender Randbereich des Schildes abtrennbar vorgesehen. Durch das Abtrennen dieses seitlichen Randbereichs wird Platz, z.B. in Form von Schlitzen, für Mundintubationen geschaffen. Der Schnuller ist von den Schläuchen frei lösbar, so dass der Schnuller in den Mund eingeführt und wieder herausgenommen werden kann, ohne dabei die Zufuhr z.B. von Nahrung durch die Schläuche unterbrechen zu müssen. Weiters können auch Mundintubationen in den Mund des Kindes eingesetzt werden, ohne das Kind durch Herausnehmen des Schnullers zu stören.

Vorteilhafterweise ist der bei der Benutzung seitlich liegende, abtrennbare Randbereich bis zu einem Intubationsloch vorgesehen. Das Intubationsloch kann so ausgebildet sein, dass es die Dimensionen von üblicher Weise eingesetzten Schläuchen aufweist, so dass ein einwandfreier Sitz des Schlauches im Schnullerschild gewährleistet ist. Auf diese Weise werden die Schläuche in der gewünschten Position gehalten, um möglichst mittig in den Mund des Frühgeborenen zugeführt zu werden und keine Verformungen im Mundbereich zu bewirken. Durch ein günstig geformtes Intubationsloch wird ein Verrutschen der Mundintubation selbst bei Bewegungen des Kopfes des Frühgeborenen weitgehend verhindert.

Es ist weiters von Vorteil, wenn der gesamte Rand des Schildes abtrennbar vorgesehen ist. Dies ist insbesondere dann wichtig, wenn das Frühgeborene sehr klein ist und die herkömmlichen Schnullerschildgrößen zu groß sind. Selbstverständlich können auch mehrere Trennstellen vorgegeben sein, so dass bei ein und demselben Schnuller verschiedene Schildgrößen zur Verfügung gestellt werden. Je nach Notwendigkeit wird der Schnullerschild für die Größe des jeweiligen Frühgeborenen zurechtgestutzt. Besonders bei sehr kleinen Frühgeborenen ist ein schmaler Schild vorteilhaft, so dass der Schnuller in einer Seitenlage des Frühgeborenen nicht aus dem Mund gedrückt wird.

Vorzugsweise sind die Schwächungsstellen als reißbare Materialverbindung ausgeführt. Dies wird z.B. durch Formen oder Stanzen von Löchern im Schnullerschild oder durch lokale Materialverdünnungen gewährleistet, so dass entlang diesen SchwächungsLinien der Schild aufgetrennt werden kann. Auf diese Weise kann sehr schnell und ohne zusätzliche Hilfsmittel die jeweils benötigte Schnullerschild-Größe zur Verfügung gestellt werden.

Von besonderem Vorteil ist es, wenn der Schnuller einteilig ausgeführt ist. Dies gewährleistet eine besonders einfache, schnelle und kostengünstige Herstellung. Insbesondere für kleine Saugergrößen ist insbesondere ein einteiliger Schnuller geeignet.

Vorzugsweise ist der Schnuller aus Silikon, Latex, Gummi, Elastomer, Thermoplast, einem thermoplastischen Elastomer und/oder einem Zweikomponentenmaterial, wie z.B. Thermoplast und Elastomer, hergestellt. Es sind auch Kombinationen von zwei oder mehreren Materialien vorstellbar. Dabei sind selbstverständlich die Anforderungen an das Material bezüglich Kleinkind-Eignung etc. zu berücksichtigen.

Besonders günstig ist es, wenn der Sauger eine einfach-symmetrische Form aufweist. Eine einfach-symmetrische Form kann z.B. durch eine Schrägstellung des Saugers in Bezug auf den Schnullerschild gegeben sein. Dadurch wird gewährleistet, dass der Schnuller in die richtige Richtung, etwa mit der Einbuchtung für die Nase nach oben gerichtet, in den Mund des Kindes eingesetzt wird, wobei auch ein späteres Umdrehen des Schnullers im Mund, das an sich bei Säuglingen häufig vorkommt, bei einer Schrägstellung des Saugers relativ gut verhindert wird. Durch die vorgegebene Ausrichtung des Schnullers im Mund des Frühgeborenen kommt es auch nicht zu einem Verdrehen von in Aussparungen und Intubationslöchern des Schildes eingesetzten Schläuchen. Zusätzlich kann aufgrund der einfach-symmetrischen Form, insbesondere der Schrägstellung des Saugers in Bezug auf den Schnullerschild, die Zunge des Kindes trainiert werden, so dass das Frühgeborene noch rascher das Saugen und Trinken lernt. Die Saugerform kann weiters verschiedene Strukturelemente aufweisen, um die Zungenund Kiefermuskulatur des Frühgeborenen zu stärken, um die Entwicklung des Frühgeborenen maximal zu fördern. Weiters kann mittels kieferorthopädisch günstigen Saugerformen Zahnfehlstellungen entgegengewirkt werden.

Vorzugsweise weist der Sauger eine Länge von 20 bis 30 mm, insbesondere ca. 20,5 mm oder ca. 23 mm, und eine maximale Breite von 10 bis 20 mm, insbesondere ca. 12,5 mm oder ca. 14,5, auf. Dabei können kleinere Sauger zur Verfügung gestellt werden, die z.B. eine Länge von ca. 20 mm und eine maximale Breite von ca. 10 mm aufweisen, die besonders für sehr kleine Frühgeborene geeignet sind. Es können auch etwas größere Sauger zur Verfügung gestellt werden, etwa mit einer Länge von ca. 30 mm und einer maximalen Breite von ca. 20 mm. Eine Möglichkeit besteht darin, drei verschiedene Saugergrößen zur Verfügung zu stellen, mit denen alle Gewichtsklassen bei Frühgeborenen abgedeckt werden: eine kleine Größe mit einer Länge von 20-21 mm und einer maximalen Breite von 12-13 mm für Säuglinge mit einem Geburtsgewicht von bis zu 1200 Gramm, eine mittlere Größe mit einer Länge von 22-23 mm und einer maximalen Breite von 14-15 mm für Säuglinge mit einem Gewicht von 1200 bis 1800 Gramm, und eine große Größe mit einer Länge von 24-25 mm und einer maximalen Breite von 16-17 mm für Säuglinge ab einem Gewicht von 1800 Gramm. Der Schnuller mit dem Sauger der großen Größe ist für den Umstieg auf "normale", handelsübliche Schnuller geeignet.

Dabei ist es vorteilhaft, wenn der Sauger entsprechend seiner Größe unterschiedlich markiert ist. Auf diese Weise kann der gewünschte Sauger schnell und ohne den Sauger abzumessen ausgewählt werden. Die Markierung kann z.B. eine in den Sauger eingearbeitete, auf den Sauger aufgedruckte oder in den Sauger eingestanzte Form oder Zahl sein.

Für ein besonders klares und eindeutiges Kennzeichnen der Sauger ist es günstig, wenn der Sauger gefärbt ist. Dadurch muss die Markierung auf dem Sauger nicht gesucht werden, sondern sie ist auf einen Blick sofort ersichtlich. Dabei ist es vorteilhaft, verschiedene, voneinander völlig unterschiedliche Farben auszuwählen, etwa gelb, grün, rot, weiß etc..

Vorzugsweise ist der Sauger aus Silikon hergestellt. Dieses Material hat sich für Sauger als besonders geeignet herausgestellt, es ist leicht zu verarbeiten, und es weist eine relativ hohe Lebensdauer auf.

Besonders bevorzugt weist der Griff ein Loch auf. Durch dieses Loch kann z.B. eine Schnur eingefädelt werden, die zur Befestigung des Schnullers an Schläuchen, Geräten bzw. am Bett des Säuglings dient. Der Griff sollte so geformt sein, dass genügend Platz für die Mund- und Nasalintubationen vorhanden bleibt: er kann z.B. scheiben-, bügel- oder stabförmig sein.

Bevorzugt steht der Griff vom Schild schräg ab. Beispielsweise weist der Griff in die Richtung weg von der Ausnehmung für die Nase, so dass der Griff dadurch keine räumliche Behinderung für Nasalintubationen darstellt und auch nicht gegen die Nase drückt.

Die Erfindung wird nachfolgend anhand von in der Zeichnung dargestellten, bevorzugten Ausführungsbeispielen noch weiter erläutert. Es zeigen:
Fig. 1 eine Draufsicht auf einen Schnuller gemäß der Erfindung;
Fig. 1a eine Teil-Schnittansicht des Schnullers gemäß der Linie A-A;
Fig. 2 eine Schnittansicht des Schnullers gemäß der Linie II-II in Fig. 1;
Fig. 3 eine Schnittansicht des Schnullers gemäß der Linie III-III in Fig. 1;
Fig. 4 eine Schnittansicht des Schnullers gemäß der Linie IV-IV in Fig. 1;
Fig. 5 eine Draufsicht auf den Schnuller gemäß Fig. 1, wobei die abtrennbaren Bereiche teilweise abgetrennt sind; und
Fig. 6 eine Unteransicht des Schnullers gemäß Fig. 1.

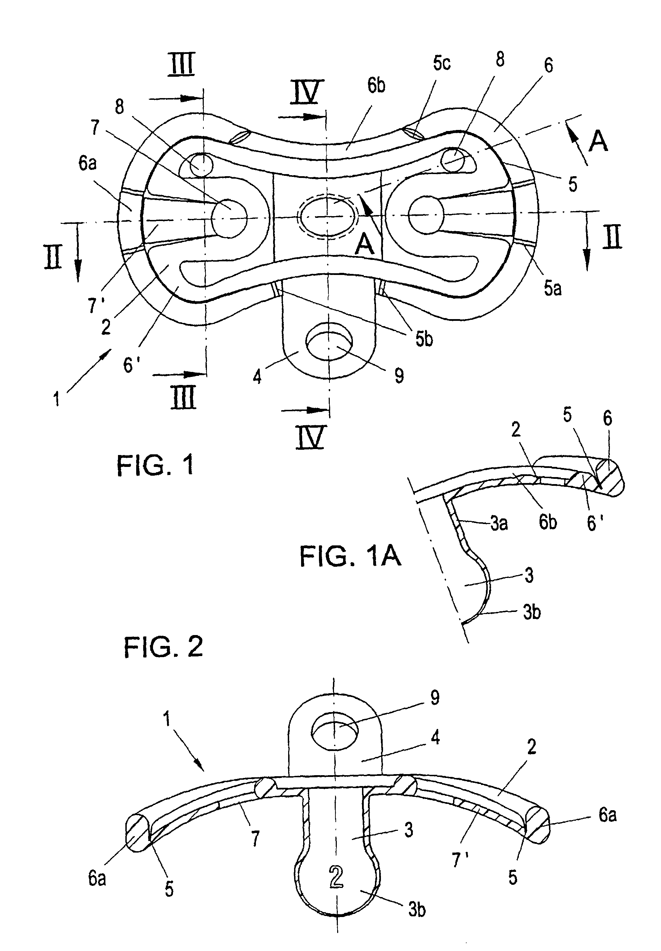

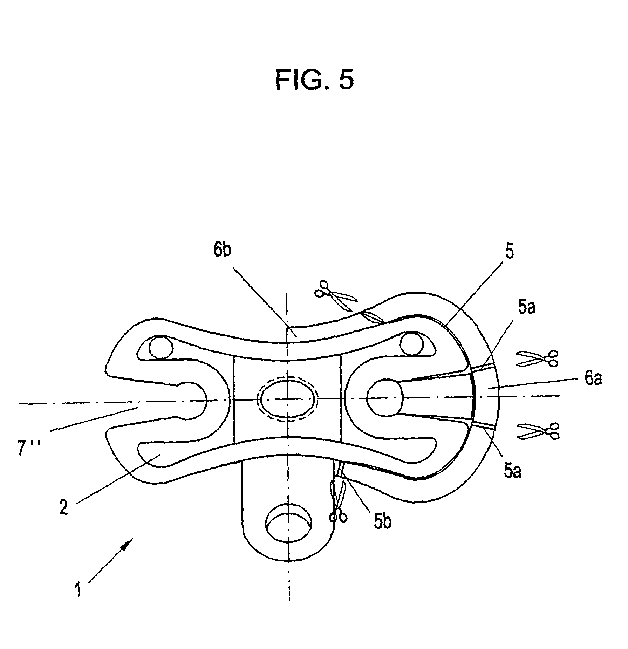

In der Zeichnung ist ein Schnuller 1, bei dem ein Schild 2 einstückig mit einem Sauger 3 und einen vom Schild 2 randseitig schräg wegstehenden Griff 4 ausgebildet ist. Der Schild 2 weist abtrennbare Bereiche 6, 6a, 6b auf, wobei zugehörige Trennstellen 5, 5a, 5b und 5c als lokale Materialverdünnungen (oder aber mit Perforationen) ausgebildet sind. Der Randbereich 6 des Schilds 2 ist als Ganzes abtrennbar, es kann aber auch lediglich z.B. ein Teil 6a des Randbereiches 6, der im seitlichen Mundwinkelbereich liegt, abgetrennt werden, wobei durch Entfernen eines Steges 7' ein Schlitz 7" freigelegt wird, um einen Zutritt zu einem Intubationsloch 7 zu gewährleisten, vgl. außer Fig. 1 und 2 auch Fig. 5. Auf diese Weise kann ein Intubationsschlauch (in der Zeichnung nicht dargestellt) durch den freigelegten Schlitz 7" in das Intubationsloch 7 eingesetzt werden.

Dadurch, dass der Griff 4 an einer Randseite schräg vom Schild 2 absteht, stellt der Griff 4 keine räumliche Behinderung für Nasalintubationen dar, die an der gegenüberliegenden Randseite vorliegt, und der Griff 4 drückt auch nicht gegen die Nase des jeweiligen Kindes.

Wird der gesamte Randbereich 6 durch Abtrennen an den Trennstellen 5 entfernt, so wird ein Schnuller 1 mit einem besonders kleinen Schild 2 erhalten, vgl. Fig. 5, linke Seite, um eine Verwendung für Frühgeborene, die sehr klein sind, zu ermöglichen. Werden die Trennstellen 5a im seitlichen Mundwinkelbereich 6a aufgebrochen, so werden die Schlitze 7" freigelegt, um einen Zutritt zu den Intubationslöchern 7 zu gewährleisten. Diese Intubationslöcher 7 sind kreisförmig, so dass jeweils ein passender, im Querschnitt runder Schlauch darin so positioniert werden kann, dass ein Verrutschen verhindert wird. Durch Abtrennen des konkaven, bogenförmigen Randbereichs 6b wird der Bereich um die Nase weitgehend freigelegt, was insbesondere für Nasalintubationen günstig ist.

Weiters sind Atmungslöcher 8 im Schild 2 vorgesehen, die - falls der Schnuller 1 vom Säugling verschluckt wird und in der Mundhöhle bzw. im Rachenbereich steckenbleibt - zusätzliche Luftzutrittmöglichkeiten gewährleisten, d.h. Notatmungsöffnungen bilden und somit ein Ersticken des Säuglings verhindern.

Der Griff 4 weist ein Loch 9 auf, durch das eine Schnur oder dergleichen gefädelt werden kann, die zur Befestigung des Schnullers 1 an Geräten, Schläuchen bzw. am Bett des Säuglings dient.

In Fig. 1a ist die Trennstelle 5 deutlich sichtbar, wobei auch ersichtlich ist, dass der Randbereich 6 im Mundwinkelbereich dicker ausgebildet ist als ein weiter innen liegender, nicht abtrennbarer Randbereich 6' (und auch als der äußere, konkave Randbereich 6b im Nasenbereich).

Der Sauger 3 weist einen Schaftteil 3a und einen Kopfteil 3b auf.

In Fig. 2 ist der Schnuller 1 von Fig. 1 im Schnitt gezeigt, wobei erkennbar ist, dass der Schild 2 gekrümmt ausgeführt ist, so dass er sich optimal an den Mundbereich des Säuglings anlegt. Der Griff 4 mit dem Loch 9 steht an einer Längsseite des Schildes schräg von diesem ab. Der Sauger 3 ist z.B. mit einer Zahl "2" markiert, was der Angabe der Größe des Saugers 3 dient. Anstelle einer Ziffer könnte der gesamte Sauger 3 bzw. ein Teil, z.B. der Kopfteil 3b, spezifisch gefärbt sein. Auf diese Weise ist gewährleistet, dass ein Schnuller mit der für das Neugeborene richtigen Saugergröße schnell gefunden wird. Weiters sind die Intubationslöcher 7, der dickere Mundwinkel-Randbereich 6a und die Trennstelle 5 zu sehen.

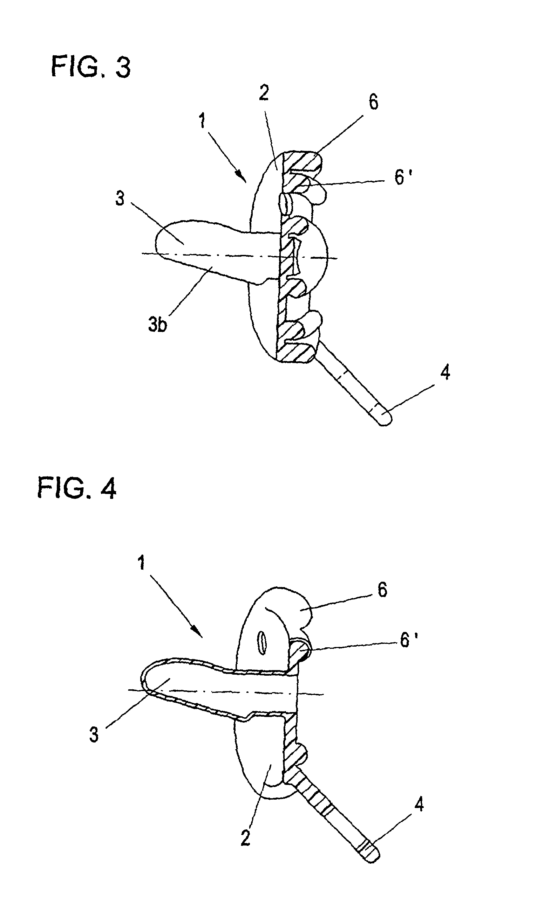

In den Fig. 3 und 4 ist ebenfalls erkennbar, dass der Randbereich 6 im Mundwinkelbereich dicker ausgebildet ist als der innerhalb davon liegende nicht abtrennbare Randbereich 6'. Aus diesen Fig. 3 und 4 ist weiters in Verbindung mit Fig. 1 und 2 ersichtlich, dass der Sauger 3 bzw. Saugerkopf 3b eine einfach-symmetrische Form aufweist, wobei der Saugerkopf 3b auf der einen Seite flach und auf der anderen Seite abgerundet ausgebildet ist.

Fig. 4 zeigt einen Schnitt gemäß der Linie IV-IV in Fig. 1, wobei der konkave Bereich 6b des Schildes 2 abgetrennt wurde. Der innere Randbereich 6' ist (im Schnitt) ersichtlich, ebenso ist der dahinterliegende, nicht abgetrennte, dickere, abtrennbare Randbereich 6 gezeigt.

Fig. 5 zeigt eine Draufsicht des vorliegenden Schnullers 1, wobei in dessen linken Hälfte die abtrennbaren Bereiche 6, 6a, 6b bereits abgetrennt wurden, wogegen diese Bereiche in der rechten Hälfte noch am Schild 2 vorhanden sind. Auf der linken Seite ist der freigelegte Schlitz 7" gezeigt. Die Trennstelle 5 ist durch eine stark gezeichnete Linie dargestellt, und bei den Trennstellen 5a, 5b sowie 5c (für den abtrennbaren Bereich 6b) ist mit Scheren-Zeichen angedeutet, dass hier ein Abtrennen mit Hilfe einer Schere, eines Messers etc. möglich ist.

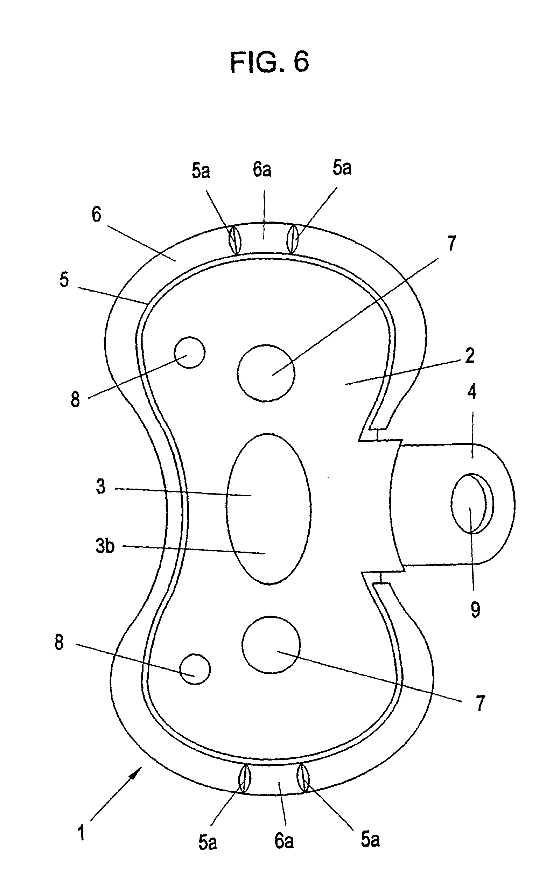

In Fig. 6 ist die Saugerseite des Schnullers 1 dargestellt, wobei auch an dieser Seite des Schildes 2 die Trennstellen 5 über den gesamten Randbereich 6 sowie die Trennstellen 5a in den seitlichen Mundwinkelbereichen 6a erkennbar sind. Im Übrigen ist ersichtlich, dass die Saugerseite des Schildes 2 - die bei Benutzung des Schnullers 1 im Mundbereich des Frühgeborenen anliegt
- glatt ausgeführt ist, sieht man von Intubationslöchern 7 und den Atmungslöchern 8 ab.

## Patentansprüche

1. Schnuller (1) für Frühgeborene mit einem Schild (2, 2a), an dem ein Sauger (3) sowie vorzugsweise ein Griff (4) angebracht ist, und der mit zumindest einer Ausnehmung für die Nase versehen ist, **dadurch gekennzeichnet, dass** der Schild (2, 2a) abtrennbare Bereiche (6, 6a, 6b) aufweist, denen als Schwächungsstellen oder Markierungen definierte, vorgegebene Trennstellen (5, 5a, 5b, 5c) zugeordnet sind.

2. Schnuller (1) nach Anspruch 1, **dadurch gekennzeichnet, dass** ein bei der Benutzung im Nasenbereich liegender Randbereich (6b) des Schildes (2) abtrennbar vorgesehen ist.

3. Schnuller (1) nach Anspruch 1 oder 2, **dadurch gekennzeichnet, dass** ein bei der Benutzung seitlich, im Mundwinkelbereich, liegender Randbereich (6a) des Schildes (2, 2a) abtrennbar vorgesehen ist.

4. Schnuller (1) nach Anspruch 3, **dadurch gekennzeichnet, dass** der bei der Benutzung seitlich liegende, abtrennbare Randbereich (6a) bis zu einem Intubationsloch (7) vorgesehen ist.

5. Schnuller (1) nach einem der Ansprüche 1 bis 4, **dadurch gekennzeichnet, dass** der gesamte Rand (6) des Schildes (2, 2a) abtrennbar vorgesehen ist.

6. Schnuller (1) nach einem der Ansprüche 1 bis 5, **dadurch gekennzeichnet, dass** die Schwächungsstellen (5, 5a, 5b, 5c) als reißbare Materialverbindung ausgeführt sind.

7. Schnuller (1) nach einem der Ansprüche 1 bis 6, **dadurch gekennzeichnet, dass** der Schnuller (1) einteilig ausgeführt ist.

8. Schnuller (1) nach einem der Ansprüche 1 bis 7, **dadurch gekennzeichnet, dass** der Schnuller (1) aus Silikon, Latex, Gummi, Elastomer, Thermoplast einem thermoplastischen Elastomer und/oder einem Zweikomponentenmaterial ist.

9. Schnuller (1) nach einem der Ansprüche 1 bis 8, **dadurch gekennzeichnet, dass** der Sauger (3) eine einfach-symmetrische Form aufweist.

10. Schnuller (1) nach einem der Ansprüche 1 bis 9, **dadurch gekennzeichnet, dass** der Sauger (1) eine Länge von 20 bis 30 mm, insbesondere ca. 20,5 mm oder ca. 23 mm, und eine maximale Breite von 10 bis 20 mm, insbesondere ca. 12,5 mm oder ca. 14,5 mm aufweist.

11. Schnuller (1) nach einem der Ansprüche 1 bis 10, **dadurch gekennzeichnet, dass** der Sauger (3) entsprechend seiner Größe unterschiedlich markiert ist.

12. Schnuller (1) nach Anspruch 11, **dadurch gekennzeichnet, dass** der Sauger (3) gefärbt ist.

13. Schnuller (1) nach einem der Ansprüche 1 bis 12, **dadurch gekennzeichnet, dass** der Sauger (3) aus Silikon hergestellt ist.

14. Schnuller (1) nach einem der Ansprüche 1 bis 13, **dadurch gekennzeichnet, dass** der Griff (4) ein Loch (9) aufweist.

15. Schnuller (1) nach Anspruch 14, **dadurch gekennzeichnet, dass** der Griff (4) vom Schild (2, 2a) schräg absteht.

## Claims

1. A pacifier (1) for premature babies, comprising a shield (2, 2a) on which a nipple (3) as well as, preferably, a grip (4) is provided, and which has at least one recess for the nose, **characterised in that** the shield (2, 2a) includes severable portions (6, 6a, 6b) having associated pre-determined severing sites (5, 5a, 5b, 5c) defined as weakened sites or markings.

2. A pacifier (1) according to claim 1, **characterised in that** a rim region (6b) of the shield (2) which, when in use, is located in the nose region, is provided to be severable.

3. A pacifier (1) according to claim 1 or 2, **characterised in that** a rim region (6a) of the shield (2, 2a) which, when in use, is laterally located in the region of the corners of the mouth, is provided to be severable.

4. A pacifier (1) according to claim 3, **characterised in that** the severable rim region (6a) which, when in use, is laterally located, is provided as far as to an intubation hole (7).

5. A pacifier (1) according to any one of claims 1 to 4, **characterised in that** the entire rim (6) of the shield (2, 2a) is provided to be severable.

6. A pacifier (1) according to any one of claims 1 to 5, **characterised in that** the weakened sites (5, 5a, 5b, 5c) are made as tearable material connections.

7. A pacifier (1) according to any one of claims 1 to 6, **characterised in that** the pacifier (1) is made in one piece.

8. A pacifier (1) according to any one of claims 1 to 7, **characterised in that** the pacifier (1) is made of silicone, latex, rubber, an elastomer, a thermoplastic material, a thermoplastic elastomer and/or a two-component material.

9. A pacifier (1) according to any one of claims 1 to 8, **characterised in that** the nipple (3) has a single-symmetrical shape.

10. A pacifier (1) according to any one of claims 1 to 9, **characterised in that** the nipple (3) has a length of from 20 to 30 mm, in particular approximately 20.5 mm or approximately 23 mm, and a maximal width of from 10 to 20 mm, in particular approximately 12.5 mm or approximately 14.5 mm.

11. A pacifier (1) according to any one of claims 1 to 10, **characterised in that** the nipple (3) is differently marked according to size.

12. A pacifier (1) according to claim 11, **characterised in that** the nipple (3) is colored.

13. A pacifier (1) according to any one of claims 1 to 12, **characterised in that** the nipple (3) is made of silicone.

14. A pacifier (1) according to any one of claims 1 to 13, **characterised in that** the grip (4) has a hole (9).

15. A pacifier (1) according to claim 14, **characterised in that** the grip (4) obliquely projects from the shield (2, 2a).

## Revendications

1. Sucette (1) pour prématuré comprenant une plaque (2, 2a), sur laquelle sont aménagées une tétine (3) ainsi que de préférence une poignée (4), et qui est dotée d'au moins un évidement pour le nez, **caractérisé en ce que** la plaque (2, 2a) comprend des zones (6, 6a, 6b) détachables, auxquelles sont associées, en tant que points d'atténuation ou marques, des points de séparation (5, 5a, 5b, 5c) prédéterminés et définis.

2. Sucette (1) selon la revendication 1, **caractérisée en ce qu'**une zone de délimitation (6b) de la plaque (2), située lors de l'utilisation dans la zone du nez, est prévue de manière à pouvoir être détachée.

3. Sucette (1) selon la revendication 1 ou 2, **caractérisée en ce qu'**une zone de délimitation (6a) de la plaque (2, 2a), située lors de l'utilisation latéralement, dans la zone de la commissure des lèvres, est prévue de manière à pouvoir être détachée.

4. Sucette (1) selon la revendication 3 , **caractérisée en ce que** la zone de délimitation (6a) détachable, située latéralement lors de l'utilisation, est prévue jusqu'à un orifice d'intubation (7).

5. Sucette (1) selon l'une quelconque des revendications 1 à 4, **caractérisée en ce que** l'ensemble du bord (6) de la plaque (2, 2a) est prévue de manière à pouvoir être détachée.

6. Sucette (1) selon l'une quelconque des revendications 1 à 5, **caractérisée en ce que** les points d'atténuation (5, 5a, 5b, 5c) sont réalisés en tant que composition de matériau déchirable.

7. Sucette (1) selon l'une quelconque des revendications 1 à 6, **caractérisée en ce que** la sucette (1) est réalisée en une seule pièce.

8. Sucette (1) selon l'une quelconque des revendications 1 à 7, **caractérisée en ce que** la sucette (1) est en silicone, en latex, en caoutchouc, en élastomère, en matière thermoplastique, en élastomère thermoplastique et/ou en matériau à deux composants.

9. Sucette (1) selon l'une quelconque des revendications 1 à 8, **caractérisée en ce que** la tétine (3) présente une forme symétrique simple.

10. Sucette (1) selon l'une quelconque des revendications 1 à 9, **caractérisée en ce que** la tétine (1) présente une longueur comprise entre 20 et 30 mm, en particulier environ 20,5 mm ou environ 23 mm, et une largeur maximale comprise entre 10 et 20 mm, en particulier environ 12,5 mm ou environ 14,5 mm.

11. Sucette (1) selon l'une quelconque des revendications 1 à 10, **caractérisée en ce que** la tétine (3) présente une marque différente en fonction de sa taille.

12. Sucette (1) selon la revendication 11, **caractérisée en ce que** la tétine (3) est colorée.

13. Sucette (1) selon l'une quelconque des revendications 1 à 12, **caractérisée en ce que** la tétine (3) est fabriquée en silicone.

14. Sucette (1) selon l'une quelconque des revendications 1 à 13, **caractérisée en ce que** la poignée (4) comprend un orifice (9).

15. Sucette (1) selon la revendication 14, **caractérisée en ce que** la poignée (4) est écartée en biais de la plaque (2, 2a).
